(19) Europäisches Patentamt
European Patent Office
Office européen des brevets

(11) Veröffentlichungsnummer: **0 591 767 A1**

(12) **EUROPÄISCHE PATENTANMELDUNG**

(21) Anmeldenummer: **93115264.9**

(51) Int. Cl.[5]: **C07D 253/07**, A01N 43/707

(22) Anmeldetag: **22.09.93**

(30) Priorität: **05.10.92 DE 4233338**

(43) Veröffentlichungstag der Anmeldung:
**13.04.94 Patentblatt 94/15**

(84) Benannte Vertragsstaaten:
**BE CH DE ES FR GB IT LI NL**

(71) Anmelder: **BAYER AG**
**D-51368 Leverkusen(DE)**

(72) Erfinder: **Kirsten, Rolf, Dr.**
**Carl-Langhans-Strasse 27**
**D-40789 Monheim(DE)**
Erfinder: **Santel, Hans-Joachim, Dr.**
**Grünstrasse 9a**
**D-51371 Leverkusen(DE)**
Erfinder: **Lürssen, Klaus, Dr.**
**August-Kierspel-Strasse 145**
**D-51469 Bergisch Gladbach(DE)**
Erfinder: **Schmidt, Robert R., Dr.**
**Im Waldwinkel 110**
**D-51467 Bergisch Gladbach(DE)**

(54) **Sulfonylierte Triazincarbonsäureamide.**

(57) Die Erfindung betrifft neue sulfonylierte Triazincarbonsäureamide der Formel (I)

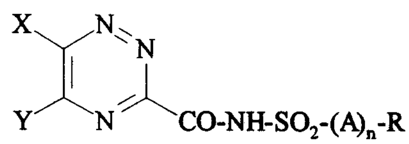

(I)

in welcher

- n für die Zahlen 0 oder 1 steht,
- A für Sauerstoff, Methylen oder die Gruppierungen NH oder N-Alkyl steht,
- R für jeweils gegebenenfalls substituiertes Aryl oder Heteroaryl steht sowie
- X und Y gleich oder verschieden sind und unabhängig voneinander für Wasserstoff, Halogen oder für jeweils gegebenenfalls substituiertes Alkyl, Alkoxy, Alkylthio, Aryl oder Heteroaryl stehen,

sowie Salze von Verbindungen der Formel (I), Verfahren zur Herstellung der neuen Wirkstoffe und deren Verwendung als Herbizide.

EP 0 591 767 A1

Die Erfindung betrifft neue sulfonylierte Triazincarbonsäureamide, ein Verfahren zu ihrer Herstellung und ihre Verwendung als Herbizide.

Es ist bereits bekannt, daß bestimmte sulfonylierte Azincarbonsäureamide herbizide Eigenschaften aufweisen (vgl. EP-A 353640, EP-A 444286). Die herbizide Wirksamkeit dieser Verbindungen ist jedoch nicht immer ganz zufriedenstellend.

Es wurden nun die neuen sulfonylierten Triazincarbonsäureamide der allgemeinen Formel (I)

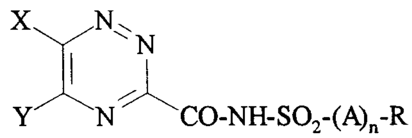

(I)

in welcher

n für die Zahlen 0 oder 1 steht,

A für Sauerstoff, Methylen oder die Gruppierungen NH oder N-Alkyl steht,

R für jeweils gegebenenfalls substituiertes Aryl oder Heteroaryl steht sowie

X und Y gleich oder verschieden sind und unabhängig voneinander für Wasserstoff, Halogen oder für jeweils gegebenenfalls substituiertes Alkyl, Alkoxy, Alkylthio, Aryl oder Heteroaryl stehen,

sowie Salze von Verbindungen der Formel (I) gefunden.

Man erhält die neuen sulfonylierten Triazincarbonsäureamide der allgemeinen Formel (I), wenn man Triazincarbonsäuren der allgemeinen Formel (II)

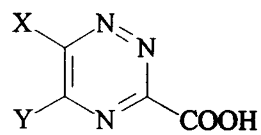

(II)

in welcher

X und Y die oben angegebene Bedeutung haben,

oder reaktionsfähige Derivate von Verbindungen der Formel (II)

mit Sulfonsäureamiden der allgemeinen Formel (III)

$H_2N-SO_2-(A)_n-R$ (III)

in welcher

n, A und R die oben angegebene Bedeutung haben,

oder mit reaktionsfähigen Derivaten von Verbindungen der Formel (III)

gegebenenfalls in Gegenwart von Reaktionshilfsmitteln und gegebenenfalls in Gegenwart von Verdünnungsmitteln umsetzt und gegebenenfalls die so erhaltenen Produkte nach üblichen Methoden in Salze überführt.

Die neuen sulfonylierten Triazincarbonsäureamide der allgemeinen Formel (I) zeichnen sich durch starke herbizide Wirksamkeit aus.

Gegenstand der Erfindung sind vorzugsweise Verbindungen der Formel (I), in welcher

n für die Zahlen 0 oder 1 steht,

A für Sauerstoff Methylen oder die Gruppierungen NH oder N-($C_1$-$C_4$-Alkyl) steht,

R für den Rest

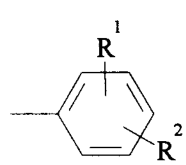

steht, worin

$R^1$ und $R^2$ gleich oder verschieden sind und für Wasserstoff, Fluor, Chlor, Brom, Iod, Cyano, Nitro, Carboxy, $C_1$-$C_6$-Alkyl (welches gegebenenfalls durch Fluor, Chlor, Brom, Cyano, Carboxy, $C_{-1}$-$C_4$-Alkoxycarbonyl, $C_1$-$C_4$-Alkylamino-carbonyl, Di-($C_1$-$C_4$-alkyl)-amino-carbonyl, Hydroxy, $C_1$-$C_4$-Alkoxy, Formyloxy, $C_1$-$C_4$-Alkyl-carbonyloxy, $C_1$-$C_4$-Alkoxycarbonyloxy, $C_1$-$C_4$-Alkylamino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-aminosulfonyl, $C_3$-$C_6$-Cycloalkyl oder Phenyl substituiert ist), für $C_2$-$C_6$-Alkenyl (welches gegebenenfalls durch Fluor, Chlor, Brom, Cyano, $C_1$-$C_4$-Alkoxycarbonyl, Carboxy oder Phenyl substituiert ist), für $C_2$-$C_6$-Alkinyl (welches gegebenenfalls durch Fluor, Chlor, Brom, Cyano, $C_1$-$C_4$-Alkoxycarbonyl, Carboxy oder Phenyl substituiert ist), für $C_1$-$C_4$-Alkoxy (welches gegebenenfalls durch Fluor, Chlor, Brom, Cyano, Carboxy, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist), für $C_1$-$C_4$-Alkylthio (welches gegebenenfalls durch Fluor, Chlor, Brom, Cyano, Carboxy, $C_1$-$C_4$-Alkoxy-carbonyl, $C_1$-$C_4$-Alkylthio, $C_1$-$C_4$-Alkylsulfinyl oder $C_1$-$C_4$-Alkylsulfonyl substituiert ist), für $C_3$-$C_6$-Alkenyloxy (welches gegebenenfalls durch Fluor, Chlor, Brom, Cyano oder $C_1$-$C_4$-Alkoxycarbonyl substituiert ist), für $C_2$-$C_6$-Alkenylthio (welches gegebenenfalls durch Fluor, Chlor, Brom, Cyano, Nitro, $C_1$-$C_3$-Alkylthio oder $C_1$-$C_4$-Alkoxycarbonyl substituiert ist), $C_3$-$C_6$-Alkinyloxy, $C_3$-$C_6$-Alkinylthio oder für den Rest $-S(O)_p-R^3$ stehen, wobei

p für die Zahlen 0, 1 oder 2 steht und

$R^3$ für $C_1$-$C_4$-Alkyl(welches gegebenenfalls durch Fluor, Chlor, Brom, Cyano oder $C_1$-$C_4$-Alkoxy-carbonyl substituiert ist), $C_3$-$C_6$-Alkenyl, $C_3$-$C_6$-Alkinyl, $C_1$-$C_4$-Alkoxy, $C_1$-$C_4$-Alkoxy-$C_1$-$C_4$-alkylamino, $C_1$-$C_4$-Alkylamino, Di-($C_1$-$C_4$-alkyl)-amino oder für den Rest $-NHOR^4$ steht, wobei

$R^4$ für $C_1$-$C_6$-Alkyl (welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist), für $C_3$-$C_6$-Alkenyl (welches gegebenenfalls durch Fluor, Chlor oder Brom substituiert ist), $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl (welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist), für Benzhydryl oder für Phenyl (welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist) steht,

$R^1$ und $R^2$ weiterhin für Phenyl oder Phenoxy, für $C_1$-$C_4$-Alkylcarbonylamino, $C_1$-$C_4$-Alkoxycarbonylamino, $C_1$-$C_4$-Alkylamino-carbonylamino, Di-($C_1$-$C_4$-alkyl)-amino-carbonylamino, oder für den Rest $-CO-R^5$ stehen, wobei

$R^5$ für $C_1$-$C_6$-Alkyl, $C_1$-$C_6$-Alkoxy, (welches gegebenenfalls durch Fluor, Chlor, Methoxy oder Ethoxy substituiert ist), $C_3$-$C_6$-Cycloalkoxy, $C_3$-$C_6$-Alkenyloxy, $C_1$-$C_4$-Alkylthio, Amino, $C_1$-$C_4$-Alkylamino, $C_1$-$C_4$-Alkoxyamino, $C_1$-$C_4$-Alkoxy-$C_1$-$C_4$-alkyl-amino oder Di-($C_1$-$C_4$-alkyl)-amino steht (welche gegebenenfalls durch Fluor und/oder Chlor substituiert sind),

$R^1$ und $R^2$ weiterhin für $C_1$-$C_4$-Alkylsulfonyloxy, Di-($C_1$-$C_4$-alkyl)aminosulfonylamino oder für den Rest $-CH=N-R^6$ stehen, wobei

$R^6$ für gegebenenfalls durch Fluor, Chlor, Cyano, Carboxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl, für gegebenenfalls durch Fluor und/oder Chlor substituiertes $C_1$-$C_6$-Alkoxy, $C_3$-$C_6$-Alkenoxy, $C_3$-$C_6$-Alkinoxy oder Benzyloxy, für Amino, $C_1$-$C_4$-Alkylamino, Di-($C_1$-$C_4$-alkyl)-amino, Phenylamino, $C_1$-$C_4$-Alkyl-carbonyl-amino, $C_1$-$C_4$-Alkoxycarbonylamino, $C_1$-$C_4$-Alkyl-sulfonylamino oder für gegebenenfalls durch Fluor, Chlor, Brom oder Methyl substituiertes Phenylsulfonylamino steht,

R weiter für den Rest

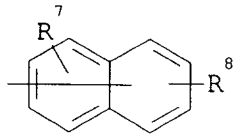

steht, worin

R⁷ und R⁸ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist) oder $C_1$-$C_4$-Alkoxy (welches gegebenenfalls durch Fluor und/oder Chlor substituiert ist), stehen;

R weiter für den Rest

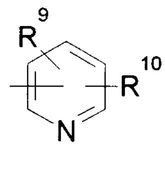

steht, worin

$R^9$ und $R^{10}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_2$-$C_4$-Alkenyl (welches gegebenenfalls durch Fluor und/oder Chlor substituiert ist),$C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), sowie für Di-($C_1$-$C_4$-alkyl)-aminosulfonyl, $C_1$-$C_4$-Alkoxy-carbonyl, Dimethylamino-carbonyl oder Dioxolanyl stehen;

R weiter für den Rest

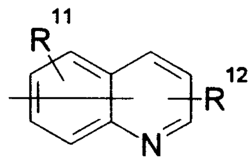

steht, worin

$R^{11}$ und $R^{12}$ gleich oder verschieden sind und für Wasserstoff, Fluor, Chlor, Brom, $C_1$-$C_4$-Alkyl (welches gegebenenfalls durch Fluor und/oder Brom substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), oder für Di-($C_1$-$C_4$-alkyl)-aminosulfonyl stehen;

R weiter für den Rest

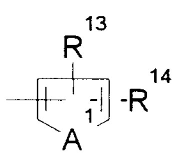

steht, worin

$R^{13}$ und $R^{14}$ gleich oder verschieden sind und für Wasserstoff, Fluor, Chlor, Brom, Cyano, Nitro, $C_1$-$C_4$-Alkyl (welches gegebenenfalls durch Fluor, Chlor, $C_1$-$C_4$-Alkoxy und/oder $C_1$-$C_4$-Halogenalkoxy substituiert ist), $C_1$-$C_4$-Alkoxy (welches gegebenenfalls durch Fluor und/oder Chlor substituiert ist), $C_1$-$C_4$-Alkylthio, $C_1$-$C_4$-Alkylsulfinyl oder $C_1$-$C_4$-Alkylsul-

fonyl (welches gegebenenfalls durch Fluor und/oder Chlor substituiert ist), Di-($C_1$-$C_4$-alkyl)-amino-sulfonyl oder $C_1$-$C_4$-Alkoxy-carbonyl stehen, und

$A^1$ für Sauerstoff, Schwefel oder die Gruppierung N-$Z^1$ steht, wobei

$Z^1$ für Wasserstoff, $C_1$-$C_4$-Alkyl (welches gegebenenfalls durch Fluor, Chlor, Brom oder Cyano substituiert ist), $C_3$-$C_6$-Cycloalkyl, Benzyl, Phenyl (welches gegebenenfalls durch Fluor, Chlor, Brom oder Nitro substituiert ist), $C_1$-$C_4$-Alkylcarbonyl, $C_1$-$C_4$-Alkoxy-carbonyl oder Di-($C_1$-$C_4$-alkyl)-aminocarbonyl steht;

R weiter für den Rest

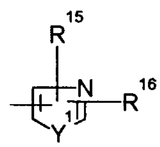

steht, worin

$R^{15}$ und $R^{16}$ gleich oder verschieden sind und für Wasserstoff, $C_1$-$C_4$-Alkyl, Halogen, $C_1$-$C_4$-Alkoxycarbonyl, $C_1$-$C_4$-Alkoxy oder $C_1$-$C_4$-Halogenalkoxy stehen,

$Y^1$ für Schwefel oder die Gruppierung N-$R^{17}$ steht, wobei

$R^{17}$ für Wasserstoff oder $C_1$-$C_4$-Alkyl steht;

R weiter für den Rest

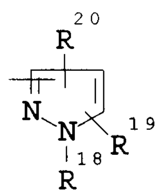

steht, worin

$R^{18}$ für Wasserstoff $C_1$-$C_4$-Alkyl, Phenyl oder (Iso)Chinolinyl steht,

$R^{19}$ für Wasserstoff Halogen, Cyano, Nitro, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), Dioxolanyl oder $C_1$-$C_4$-Alkoxy-carbonyl steht und

$R^{20}$ für Wasserstoff Halogen oder $C_1$-$C_4$-Alkyl steht;

R weiter für den Rest

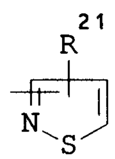

steht, worin

$R^{21}$ für Wasserstoff Halogen, $C_1$-$C_4$-Alkyl oder $C_1$-$C_4$-Alkoxy-carbonyl steht; sowie

X und Y gleich oder verschieden sind und unabhängig voneinander für Wasserstoff Fluor, Chlor, Brom oder für jeweils gegebenenfalls durch Fluor, Chlor oder Brom substituiertes $C_1$-$C_4$-Alkyl, $C_1$-$C_4$-Alkoxy oder $C_1$-$C_4$-Alkylthio, oder für jeweils gegebenenfalls durch Fluor, Chlor, Brom $C_1$-$C_4$-Alkyl, Trifluormethyl, $C_1$-$C_4$-Alkoxy, Difluormethoxy, Trifluoromethoxy, Difluormethylthio, Trifluormethylthio oder $C_1$-$C_4$-Alkylthio substituiertes Phenyl, Naphthyl, Pyridyl, Furyl oder Thienyl stehen.

Die Erfindung betrifft weiter vorzugsweise Salze von Verbindungen der Formel (I)

a) mit Protonensäuren, wie z.B. Salzsäure, Schwefelsäure, Phosphorsäure, Methansulfonsäure, Benzol- oder p-Toluolsulfonsäure, oder Naphthalin-mono- oder -di-sulfonsäuren, oder

b) mit Basen, wie z.B. Natrium-, Kalium- oder Calcium-hydroxid, -hydrid, -amid oder -carbonat, Natrium- oder Kalium-$C_1$-$C_4$-alkanolaten, Ammoniak, $C_1$-$C_4$-Alkylaminen, Di-($C_1$-$C_4$-alkyl)-aminen oder Tri-($C_1$-$C_4$-alkyl)-aminen.

Die Erfindung betrifft insbesondere Verbindungen der Formel (I), in welcher

n für die Zahl 0 steht,

R für den Rest

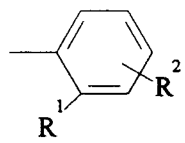

steht, worin

$R^1$ für Fluor, Chlor, Brom, Cyano, Nitro, Carboxy, Methyl, Trifluormethyl, Methoxy, Ethoxy, Difluormethoxy, Trifluormethoxy, $C_1$-$C_3$-Alkylthio, $C_1$-$C_3$-Alkylsulfinyl, $C_1$-$C_3$-Alkylsulfonyl, Dimethylaminosulfonyl, Diethylaminosulfonyl, N-Methoxy-N-methylaminosulfonyl, Phenyl, Phenoxy oder $C_1$-$C_3$-Alkoxycarbonyl steht und

$R^2$ für Wasserstoff, Fluor, Chlor, Brom, Methyl, Trifluormethyl oder Methoxy steht;

X für Wasserstoff, Methyl, Ethyl, Propyl, Methoxy, Ethoxy, Propoxy, Methylthio, Ethylthio, Propylthio oder Phenyl steht und

Y für Wasserstoff Methyl, Ethyl, Propyl, Methoxy, Ethoxy, Propoxy, Methylthio, Ethylthio, Propylthio oder Phenyl steht.

Die oben aufgeführten allgemeinen oder in Vorzugsbereichen aufgeführten Restedefinitionen bzw.Erläuterungen gelten für die Endprodukte und für die Ausgangs- und Zwischenprodukte entsprechend. Diese Restedefinitionen können untereinander, also auch zwischen den jeweiligen Vorzugsbereichen beliebig kombiniert werden.

Beispiele für die erfindungsgemäßen Verbindungen der Formel (I) sind in der nachstehenden Tabelle 1 aufgeführt.

Tabelle 1: Beispiele für die Verbindungen der Formel (I)

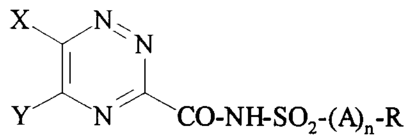

(I)

| n | A | R | X | Y |
|---|---|---|---|---|
| 0 | - | 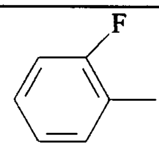 | $CH_3$ | $CH_3$ |
| 0 | - | 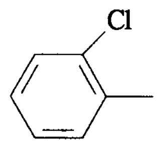 | $CH_3$ | $C_2H_5$ |
| 0 | - | 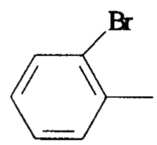 | $C_2H_5$ | $CH_3$ |
| 1 | $CH_2$ | 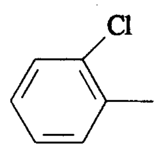 | $OCH_3$ | $CH_3$ |
| 1 | O | 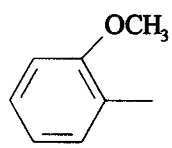 | $CH_3$ | $OC_2H_5$ |
| 1 | NH | 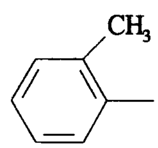 | $CH_3$ | $CH_3$ |

Tabelle 1: - Fortsetzung

| n | A | R | X | Y |
|---|---|---|---|---|
| 0 | - | 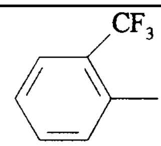 | $C_2H_5$ | $OCH_3$ |
| 0 | - | 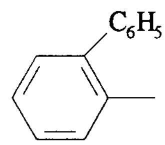 | $CH_3$ | $OC_2H_5$ |
| 0 | - | 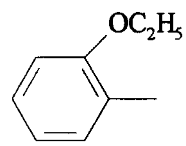 | $SCH_3$ | $CH_3$ |
| 0 | - | 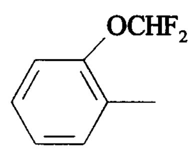 | $CH_3$ | $SC_2H_5$ |
| 0 | - | 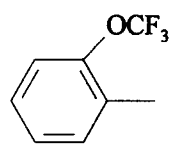 | $OCH_3$ | $OCH_3$ |
| 0 | - | 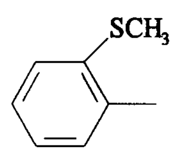 | $CH_3$ | $C_2H_5$ |
| 0 | - | 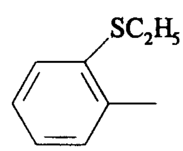 | $CH_3$ | $CH_3$ |
| 0 | - | 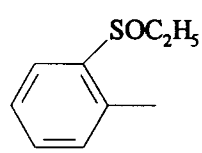 | $CH_3$ | $CH_3$ |

Tabelle 1: - Fortsetzung

| n | A | R | X | Y |
|---|---|---|---|---|
| 0 | - | 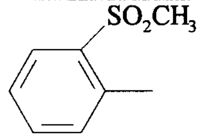 | $OCH_3$ | $CH_3$ |
| 0 | - | 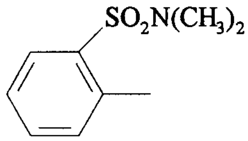 | $CH_3$ | $OCH_3$ |
| 0 | - | 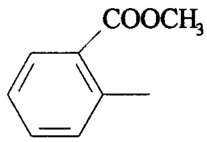 | $CH_3$ | $CH_3$ |
| 0 | - | 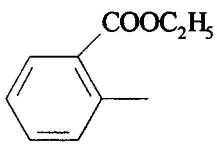 | $CH_3$ | $CH_3$ |
| 1 | $CH_2$ | 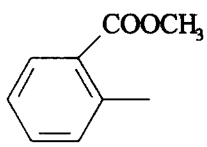 | $OCH_3$ | $OCH_3$ |
| 0 | - | 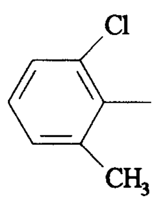 | $CH_3$ | $CH_3$ |

Verwendet man beispielsweise 5,6-Dimethyl-1,2,4-triazin-3-carbonsäurechlorid und 2-Fluor-benzolsulfonsäureamid als Ausgangsstoffe, so kann der Reaktionsablauf beim erfindungsgemäßen Verfahren durch das folgende Formelschema skizziert ert werden:

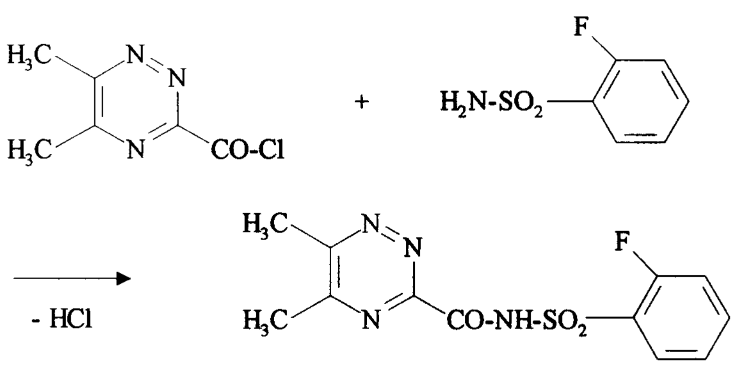

Die beim erfindungsgemäßen Verfahren zur Herstellung von Verbindungen der Formel (I) als Ausgangsstoffe zu verwendenden Triazincarbonsäuren sind durch die Formel (II) allgemein definiert.

In Formel (II) haben X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für X und Y angegeben wurden.

Die Ausgangsstoffe der Formel (II) sind bekannt und/oder können nach an sich bekannten Verfahren hergestellt werden (vgl. Heterocycles 30 (1990), 1009-1021; Liebigs Ann. Chem. 1990, 631-640; J. Org. Chem. 55 (1990), 3257-3269; Tetrahedron Lett. 21 (1980), 595-598; Tetrahedron Lett. 1975, 2901-2904; Tetrahedron Lett. 28 (1987), 6027-6030; Synthesis 1974, 351-352; Helv. Chim. Acta 38 (1955), 1560).

Anstatt der Triazincarbonsäuren der Formel (II) werden beim erfindungsgemäßen Verfahren gegebenenfalls die davon abgeleiteten Carbonsäurechloride oder davon abgeleitete Alkylester (vorzugsweise Methylester oder Ethylester), Aralkylester (vorzugsweise Benzylester) oder Arylester (vorzugsweise Phenylester, in der Phenylgruppe gegebenenfalls durch Cyano, Nitro, Chlor, Fluor, Brom und/oder Methyl substituiert) als Ausgangsstoffe ("reaktionsfähige Derivate") eingesetzt.

Man erhält die von den Verbindungen der Formel (II) abgeleiteten Carbonsäurechloride aus den entsprechenden Carbonsäuren nach üblichen Methoden, beispielsweise durch Umsetzung mit üblicherweise als "Chlorierungsmittel" verwendeten Säurechloriden, wie z.B. Phosgen, Oxalylchlorid oder Thionylchlorid, gegebenenfalls in Gegenwart von Reaktionshilfsmitteln, wie z.B. Pyridin, und gegebenenfalls in Gegenwart von Verdünnungsmitteln, wie z.B. Chloroform oder Tetrachlormethan, bei Temperaturen zwischen 0 °C und 100 °C.

Die als weitere reaktionsfähige Derivate der Verbindungen der Formel (II) genannten Ester können aus den entsprechenden Carbonsäurechloriden und geeigneten Alkoholen oder Phenolen nach üblichen Methoden, im allgemeinen durch Umsetzung in Gegenwart eines Säureakzeptors, wie z.B. Triethylamin oder Pyridin, und in Gegenwart eines Verdünnungsmittels, wie z.B. Acetonitril, bei Temperaturen zwischen 0 °C und 100 °C erhalten werden.

Man kann die genannten Ester jedoch auch direkt aus den Carbonsäuren der Formel (II) in Gegenwart von Kondensationshilfsmitteln, wie z.B. Dicyclohexylcarbodiimid, gegebenenfalls in Gegenwart von Reaktionshilfsmitteln, wie z.B. 4-Dimethylaminopyridin, und gegebenenfalls in Gegenwart von Verdünnungsmitteln, wie z.B. Methylenchlorid oder Choroform, bei Temperaturen zwischen 0 °C und 100 °C erhalten.

Die beim erfindungsgemäßen Verfahren zur Herstellung von Verbindungen der Formel (I) weiter als Ausgangsstoffe zu verwendenden Sulfonsäureamide sind durch die Formel (III) allgemein definiert.

In Formel (III) haben n, A und R vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, A und R angegeben wurden.

Die Ausgangsstoffe der Formel (III) sind bekannt und/oder können nach an sich bekannten Verfahren hergestellt werden (vgl. EP-A 7687, EP-A 23140, EP-A 23141, EP-A 23422, EP-A 35893, EP-A 48143. EP-A 51466, EP-A 64322, EP-A 70041, EP-A 44808, EP-A 44809, US-P 2929820, US-P 4282242, US-P 4348220, US-P 4372778).

Anstatt der Sulfonsäureamide der Formel (III) werden beim erfindungsgemäßen Verfahren gegebenenfalls die davon abgeleiteten Sulfonylisocyanate ("reaktionsfahige Derivate") als Ausgangsstoffe eingesetzt. Diese Verbindungen sind bekannt und/oder können nach an sich bekannten Verfahren hergestellt werden (vgl. J. Org. Chem. 31 (1966), 2658-2661; EP-A 7687; EP-A 46626; EP-A 21641; EP-A 23140; EP-A 23141; EP-A 70041; EP-A 23422, EP-A 64322; EP-A 34431; EP-A 35893; EP-A 51466, EP-A 44808; EP-A 173312; DE-OS 3132944; EP-A 87780; EP-A 271780).

Das erfindungsgemäße Verfahren wird gegebenenfalls in Gegenwart eines Reaktionshilfsmittels durchgeführt. Geeignete Reaktionshilfsmittel sind hierbei Verbindungen, mit denen Carbonsäuren in reaktionsfähige Intermediate überführt werden, die dann in situ mit nucleophilen Verbindungen, wie beispielsweise den Sulfonsäureamiden der Formel (III) zu entsprechenden Carbonsäurederivaten umgesetzt werden. Als Beispiele für derartige Reaktionshilfsmittel seien Carbonyldiimidazol und 2-Chlor-1-methyl-pyridiniumiodid genannt.

Für die Umsetzung der als reaktionsfähige Derivate von Verbindungen der Formel (II) genannten Carbonsäurechloride und Ester mit Sulfonsäureamiden der Formel (III) sind auch Säureakzeptoren, wie sie nachstehend angegeben sind, als Reaktionshilfsmittel geeignet.

Als Säureakzeptoren können bei dem erfindungsgemäßen Verfahren alle üblicherweise für derartige Umsetzungen verwendbaren Säurebindemittel eingesetzt werden. Vorzugsweise infrage kommen Alkalimetall- und Erdalkalimetallhydride, wie Lithium-, Natrium-, Kalium- und Calcium-hydrid, Alkalimetall- und Erdalkalimetall-hydroxide, wie Lithium-, Natrium-, Kalium- und Calcium-hydroxid, Alkalimetall- und Erdalkalimetall-carbonate und -hydrogencarbonate, wie Natrium- und Kalium-carbonat oder -hydrogencarbonat sowie

Calciumcarbonat, Alkalimetallacetate, wie Natrium- und Kalium-acetat, Alkalimetallalkoholate, wie Natrium- und Kalium-methylat, -ethylat, -propylat, -isopropylat, -butylat, -isobutylat und tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO).

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Die Reaktionstemperaturen können bei dem erfindungsgemäßen Verfahren in einem größeren Bereich variiert werden. Im allgemeinen arbeitet man bei Temperaturen zwischen 0 °C und 150 °C, vorzugsweise bei Temperaturen zwischen 10 °C und 100 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens können die Sulfonsäureamide der Formel (III) auch vor der Umsetzung mit den Triazincarbonsäuren der Formel (II) oder deren reaktionsfähigen Derivaten durch Behandeln mit geeigneten Säureakzeptoren, wie z.B. Natrium- oder Kalium-methanolat oder -ethanolat, in geeigneten Verdünnungsmitteln, wie z.B. Methanol oder Ethanol, in entsprechende Salze überführt werden, die dann - vorteilhaft nach Wechsel des Verdünnungsmittels - zur weiteren Umsetzung eingesetzt werden (vgl. die Herstellungsbeispiele).

Zur Durchführung des erfindungsgemäßen Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel gegebenenfalls in Gegenwart eines Reaktionshilfsmittels durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden (vgl. die Herstellungsbeispiele).

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z.B. Wasser, Methanol, Ethanol, Methylenchlorid oder Aceton, und Zugabe einer geeigneten Säure bzw. Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen Kulturen, wie z.B. Weizen, vor allem im Vorauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofopethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr,

Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuronmethyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

Herstellungsbeispiele:

Beispiel 1

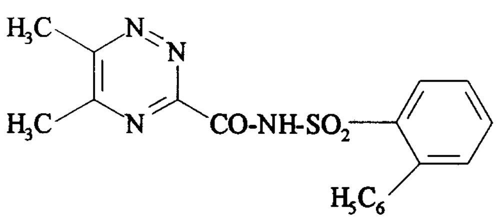

4,66 g (0,02 Mol) 2-Phenyl-benzolsulfonsäureamid werden in das Natriumsalz überführt, indem man es in 100 ml Methanol löst, 1,1 g (0,02 Mol) Natriummethanolat zufügt, 10 Minuten nachrührt und zur Trockene eindampft. Das Natriumsalz wird in 100 ml Acetonitril mit 3,62 g (0,02 Mol) 5,6-Dimethyl-1,2,4-triazin-3-carbonsäure-ethylester 15 Stunden unter Rückfluß gekocht. Der nach dem Abdestillieren des Lösungsmittels verbleibende Rückstand wird mit 100 ml 10%iger wäßriger Kaliumcarbonatlösung verrührt. Man saugt das Ungelöste ab und säuert das Filtrat mit 1N-Salzsäure an. Man extrahiert das Produkt mit Methylenchlorid, trocknet die Methylenchloridlösung über Natriumsulfat, filtriert und engt das Filtrat ein. Der Rückstand wird mit Diethylether verrührt, abgesaugt und an der Luft getrocknet.

Man erhält 1,1 g (15% der Theorie) N-(2-Phenyl-phenylsulfonyl)-5,6-dimethy-1,2,4-triazin-3-carbonsäureamid vom Schmelzpunkt 190 °C (Zers.).

Analog Beispiel 1 und entsprechend der allgemeinen Beschreibung des erfindungsgemäßen Herstellungsverfahrens können beispielsweise auch die in der nachstehenden Tabelle 2 aufgeführten Verbindungen der Formel (I) hergestellt werden.

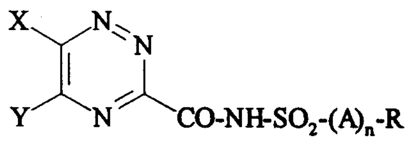

(I)

Tabelle 2: Herstellungsbeispiele für die Verbindungen der Formel (I)

| Bsp.-Nr. | n | A | R | X | Y | Physikal.Daten |
|---|---|---|---|---|---|---|
| 2 | 0 | - | 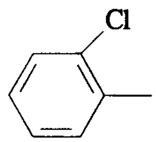 | $CH_3$ | $CH_3$ | Fp.: 118°C (Zersetz.) |
| 3 | 0 | - | 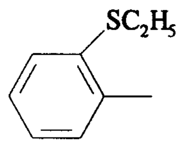 | $CH_3$ | $CH_3$ | Fp.: 65°C (Zersetz.) |
| 4 | 0 | - | 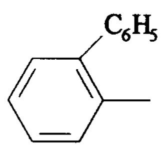 | $C_2H_5$ | $C_2H_5$ | Fp.: 68°C (Zersetz.) |
| 5 | 0 | - | 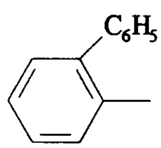 | $C_2H_5$ | $CH_3$ | Fp.: 155°C (Zersetz.) |
| 6 | 0 | - | 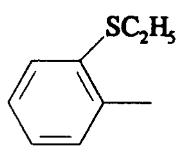 | $C_2H_5$ | $CH_3$ | Fp.: 82°C |
| 7 | 0 | - | 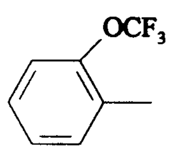 | $C_2H_5$ | $CH_3$ | Fp.: 95°C (Zersetz.) |
| 8 | 0 | - | 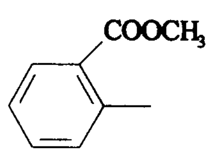 | $CH_3$ | $CH_3$ | Fp.: 124°C (Zersetz.) |

<u>Tabelle 2:</u> - Fortsetzung

| Bsp.-Nr. | n | A | R | X | Y | Physikal.Daten |
|---|---|---|---|---|---|---|
| 9 | 0 | - | 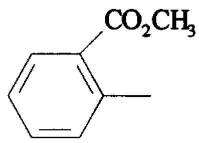 | $C_2H_5$ | $C_2H_5$ | Öl |
| 10 | 0 | - | 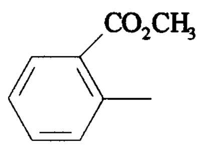 | $C_2H_5$ | $CH_3$ | |
| 11 | 0 | - | 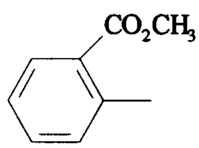 | $C_3H_7$-n | $CH_3$ | |
| 12 | 0 | - | 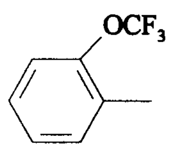 | $CH_3$ | $CH_3$ | Fp.: 80°C |
| 13 | 0 | - | 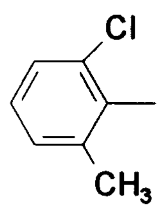 | $CH_3$ | $CH_3$ | Fp.: 187°C |
| 14 | 0 | - | 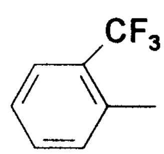 | $CH_3$ | $CH_3$ | Fp.: 93°C (Zersetz.) |
| 15 | 0 | - | 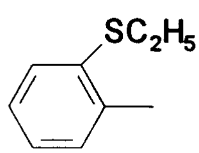 | $C_6H_5$ | $C_6H_5$ | Fp.: 143°C (Zersetz.) |

Tabelle 2: - Fortsetzung

| Bsp.-Nr. | n | A | R | X | Y | Physikal.Daten |
|---|---|---|---|---|---|---|
| 16 | 0 | - | 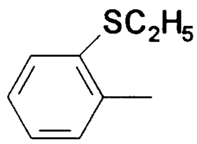 | $C_2H_5$ | $C_2H_5$ | Fp.: 124°C (Zersetz.) |
| 17 | 0 | - | 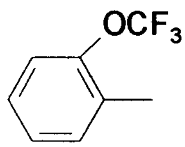 | $C_2H_5$ | $C_2H_5$ | Fp.: 62°C |
| 18 | 0 | - | 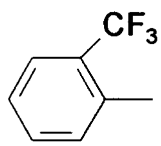 | $C_2H_5$ | $C_2H_5$ | Fp.: 73°C (Zersetz.) |
| 19 | 0 | - | 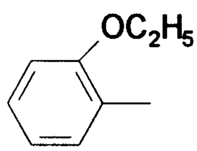 | H | $C_6H_5$ | Fp.: 193°C |
| 20 | 0 | - | 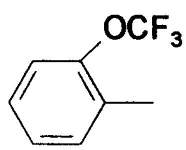 | H | $C_6H_5$ | Fp.: 216°C |
| 21 | 0 | - | 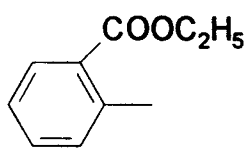 | $CH_3$ | $CH_3$ | Fp.: 82°C |

"Fp. (Zersetz.)" = Schmelzpunkt (unter Zersetzung)

Anwendungsbeispiele:

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Auftwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

O % = keine Wirkung (wie unbehandelte Kontrolle)

100 % = totale Vernichtung

In diesem Test zeigen beispielsweise die Verbindungen aus den Herstellungsbeispielen 1, 2 und 3 bei teilweise guter Verträglichkeit gegenüber Kulturpflanzen, wie z.B. Weizen, starke Wirkung gegen Unkräuter.

**Patentansprüche**

1. Sulfonylierte Triazincarbonsäureamide der allgemeinen Formel (I)

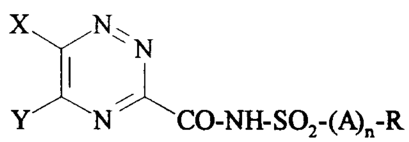

(I)

in welcher

| | |
|---|---|
| n | für die Zahlen 0 oder 1 steht, |
| A | für Sauerstoff, Methylen oder die Gruppierungen NH oder N-Alkyl steht, |
| R | für jeweils gegebenenfalls substituiertes Aryl oder Heteroaryl steht sowie |
| X und Y | gleich oder verschieden sind und unabhängig voneinander für Wasserstoff, Halogen oder für jeweils gegebenenfalls substituiertes Alkyl, Alkoxy, Alkylthio, Aryl oder Heteroaryl stehen, |

sowie Salze von Verbindungen der Formel (I).

2. Triazincarbonsäureamide der Formel (I) gemäß Anspruch 1 und deren Salze, dadurch gekennzeichnet, daß in Formel (I)

| | |
|---|---|
| n | für die Zahlen 0 oder 1 steht, |
| A | für Sauerstoff, Methylen oder die Gruppierungen NH oder N-($C_1$-$C_4$-Alkyl) steht, |
| R | für den Rest |

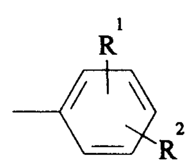

steht, worin

$R^1$ und $R^2$ gleich oder verschieden sind und für Wasserstoff, Fluor, Chlor, Brom, Iod, Cyano, Nitro, Carboxy, $C_1$-$C_6$-Alkyl (welches gegebenenfalls durch Fluor, Chlor, Brom, Cyano, Carboxy, $C_1$-$C_4$-Alkoxycarbonyl, $C_1$-$C_4$-Alkylamino-carbonyl, Di-($C_1$-$C_4$-alkyl)-amino-carbonyl, Hydroxy, $C_1$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkylamino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-aminosulfonyl, $C_3$-$C_6$-Cycloalkyl oder Phenyl substituiert ist), für $C_2$-$C_6$-Alkenyl (welches gegebenenfalls durch Fluor, Chlor, Brom, Cyano, $C_1$-$C_4$-Alkoxycarbonyl, Carboxy oder Phenyl substituiert ist), für $C_2$-$C_6$-Alkinyl (welches gegebenenfalls durch Fluor, Chlor, Brom, Cyano, $C_1$-$C_4$-Alkoxy-carbonyl, Carboxy oder Phenyl substituiert ist), für $C_1$-$C_4$-Alkoxy (welches gegebenenfalls durch Fluor, Chlor, Brom, Cyano, Carboxy, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substitu-

iert ist), für $C_1$-$C_4$-Alkylthio (welches gegebenenfalls durch Fluor, Chlor, Brom, Cyano, Carboxy, $C_1$-$C_4$-Alkoxy-carbonyl, $C_1$-$C_4$-Alkylthio, $C_1$-$C_4$-Alkylsulfinyl oder $C_1$-$C_4$-Alkylsulfonyl substituiert ist), für $C_3$-$C_6$-Alkenyloxy (welches gegebenenfalls durch Fluor, Chlor, Brom, Cyano oder $C_1$-$C_4$-Alkoxycarbonyl substituiert ist), für $C_2$-$C_6$-Alkenylthio (welches gegebenenfalls durch Fluor, Chlor, Brom, Cyano, Nitro, $C_1$-$C_3$-Alkylthio oder $C_1$-$C_4$-Alkoxycarbonyl substituiert ist), $C_3$-$C_6$-Alkinyloxy, $C_3$-$C_6$-Alkinylthio oder für den Rest $-S(O)_p-R^3$ stehen, wobei

p für die Zahlen 0, 1 oder 2 steht und

$R^3$ für $C_1$-$C_4$-Alkyl (welches gegebenenfalls durch Fluor, Chlor, Brom, Cyano oder $C_1$-$C_4$-Alkoxy-carbonyl substituiert ist), $C_3$-$C_6$-Alkenyl, $C_3$-$C_6$-Alkinyl, $C_1$-$C_4$-Alkoxy, $C_1$-$C_4$-Alkoxy-$C_1$-$C_4$-alkylamino, $C_1$-$C_4$-Alkylamino, Di-($C_1$-$C_4$-alkyl)-amino oder für den Rest $-NHOR^4$ steht, wobei

$R^4$ für $C_1$-$C_6$-Alkyl (welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkylcarbonyl, $C_1$-$C_4$-Alkoxy-carbonyl, $C_1$-$C_4$-Alkylaminocarbonyl oder Di-($C_1$-$C_4$-alkyl)-amino-carbonyl substituiert ist), für $C_3$-$C_6$-Alkenyl (welches gegebenenfalls durch Fluor, Chlor oder Brom substituiert ist), $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl (welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist), für Benzhydryl oder für Phenyl (welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist) steht,

$R^1$ und $R^2$ weiterhin für Phenyl oder Phenoxy, für $C_1$-$C_4$-Alkylcarbonylamino, $C_1$-$C_4$-Alkoxycarbonylamino, $C_1$-$C_4$-Alkylamino-carbonylamino, Di-($C_1$-$C_4$-alkyl)-amino-carbonylamino, oder für den Rest $-CO-R^5$ stehen, wobei

$R^5$ für $C_1$-$C_6$-Alkyl, $C_1$-$C_6$-Alkoxy, (welches gegebenenfalls durch Fluor, Chlor, Methoxy oder Ethoxy substituiert ist), $C_3$-$C_6$-Cycloalkoxy, $C_3$-$C_6$-Alkenyloxy, $C_1$-$C_4$-Alkylthio, Amino, $C_1$-$C_4$-Alkylamino, $C_1$-$C_4$-Alkoxyamino, $C_1$-$C_4$-Alkoxy-$C_1$-$C_4$-alkyl-amino oder Di-($C_1$-$C_4$-alkyl)-amino steht (welche gegebenenfalls durch Fluor und/oder Chlor substituiert sind),

$R^1$ und $R^2$ weiterhin für $C_1$-$C_4$-Alkylsulfonyloxy, Di-($C_1$-$C_4$-alkyl)aminosulfonylamino oder für den Rest $-CH=N-R^6$ stehen, wobei

$R^6$ für gegebenenfalls durch Fluor, Chlor, Cyano, Carboxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl, für gegebenenfalls durch Fluor und/oder Chlor substituiertes $C_1$-$C_6$-Alkoxy, $C_3$-$C_6$-Alkenoxy, $C_3$-$C_6$-Alkinoxy oder Benzyloxy, für Amino, $C_1$-$C_4$-Alkylamino, Di-($C_1$-$C_4$-alkyl)-amino, Phenylamino, $C_1$-$C_4$-Alkyl-carbonyl-amino, $C_1$-$C_4$-Alkoxycarbonylamino, $C_1$-$C_4$-Alkyl-sulfonylamino oder für gegebenenfalls durch Fluor, Chlor, Brom oder Methyl substituiertes Phenylsulfonylamino steht,

R weiter für den Rest

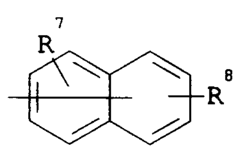

steht, worin

$R^7$ und $R^8$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist) oder $C_1$-$C_4$-Alkoxy (welches gegebenenfalls durch Fluor und/oder Chlor substituiert ist), stehen;

R weiter für den Rest

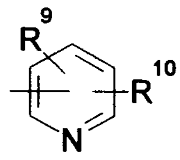

steht, worin

$R^9$ und $R^{10}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_2$-$C_4$-Alkenyl (welches gegebenenfalls durch Fluor und/oder Chlor substituiert ist),$C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), sowie für Di-($C_1$-$C_4$-alkyl)-aminosulfonyl, $C_1$-$C_4$-Alkoxy-carbonyl, Dimethylaminocarbonyl oder Dioxolanyl stehen;

R weiter für den Rest

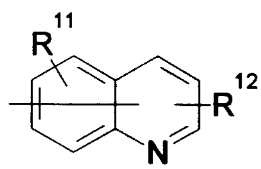

steht, worin

$R^{11}$ und $R^{12}$ gleich oder verschieden sind und für Wasserstoff, Fluor, Chlor, Brom, $C_1$-$C_4$-Alkyl (welches gegebenenfalls durch Fluor und/oder Brom substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), oder für Di-($C_1$-$C_4$-alkyl)-aminosulfonyl stehen;

R weiter für den Rest

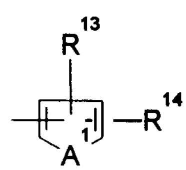

steht, worin

$R^{13}$ und $R^{14}$ gleich oder verschieden sind und für Wasserstoff, Fluor, Chlor, Brom, Cyano, Nitro, $C_1$-$C_4$-Alkyl (welches gegebenenfalls durch Fluor, Chlor, $C_1$-$C_4$-Alkoxy und/oder $C_1$-$C_4$-Halogenalkoxy substituiert ist), $C_1$-$C_4$-Alkoxy (welches gegebenenfalls durch Fluor und/oder Chlor substituiert ist), $C_1$-$C_4$-Alkylthio, $C_1$-$C_4$-Alkylsulfinyl oder $C_1$-$C_4$-Alkylsulfonyl (welches gegebenenfalls durch Fluor und/oder Chlor substituiert ist), Di-($C_1$-$C_4$-alkyl)-amino-sulfonyl oder $C_1$-$C_4$-Alkoxy-carbonyl stehen, und

$A^1$ für Sauerstoff, Schwefel oder die Gruppierung N-$Z^1$ steht, wobei

$Z^1$ für Wasserstoff, $C_1$-$C_4$-Alkyl (welches gegebenenfalls durch Fluor, Chlor, Brom oder Cyano substituiert ist), $C_3$-$C_6$-Cycloalkyl, Benzyl, Phenyl (welches gegebenenfalls durch Fluor, Chlor, Brom oder Nitro substituiert ist), $C_1$-$C_4$-Alkylcarbonyl, $C_1$-$C_4$-Alkoxy-carbonyl oder Di-($C_1$-$C_4$-alkyl)-aminocarbonyl steht;

R weiter für den Rest

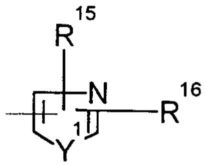

steht, worin

$R^{15}$ und $R^{16}$ gleich oder verschieden sind und für Wasserstoff, $C_1$-$C_4$-Alkyl, Halogen, $C_1$-$C_4$-Alkoxycarbonyl, $C_1$-$C_4$-Alkoxy oder $C_1$-$C_4$-Halogenalkoxy stehen,

$Y^1$ für Schwefel oder die Gruppierung N-$R^{17}$ steht, wobei

$R^{17}$ für Wasserstoff oder $C_1$-$C_4$-Alkyl steht;

R weiter für den Rest

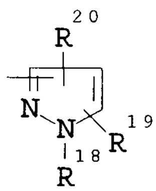

steht, worin

$R^{18}$ für Wasserstoff, $C_1$-$C_4$-Alkyl, Phenyl oder (Iso)Chinolinyl steht,

$R^{19}$ für Wasserstoff, Halogen, Cyano, Nitro, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), Dioxolanyl oder $C_1$-$C_4$-Alkoxy-carbonyl steht und

$R^{20}$ für Wasserstoff, Halogen oder $C_1$-$C_4$-Alkyl steht;

R weiter für den Rest

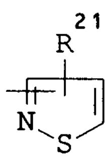

steht, worin

$R^{21}$ für Wasserstoff, Halogen, $C_1$-$C_4$-Alkyl oder $C_1$-$C_4$-Alkoxycarbonyl steht; sowie

X und Y gleich oder verschieden sind und unabhängig voneinander für Wasserstoff, Fluor, Chlor, Brom oder für jeweils gegebenenfalls durch Fluor, Chlor oder Brom substituiertes $C_1$-$C_4$-Alkyl, $C_1$-$C_4$-Alkoxy oder $C_1$-$C_4$-Alkylthio, oder für jeweils gegebenenfalls durch Fluor, Chlor, Brom, $C_1$-$C_4$-Alkyl, Trifluormethyl, $C_1$-$C_4$-Alkoxy, Difluormethoxy, Trifluormethoxy, Difluormethylthio, Trifluormethylthio oder $C_1$-$C_4$-Alkylthio substituiertes Phenyl, Naphthyl, Pyridyl, Furyl oder Thienyl stehen.

**3.** Triazincarbonsäureamide der Formel (I) gemäß Anspruch 1 und deren Salze, dadurch gekennzeichnet, daß in Formel (I)

n für die Zahl 0 steht,

R für den Rest

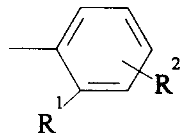

steht, worin

$R^1$ für Fluor, Chlor, Brom, Cyano, Nitro, Carboxy, Methyl, Trifluormethyl, Methoxy, Ethoxy, Difluormethoxy, Trifluormethoxy, $C_1$-$C_3$-Alkylthio, $C_1$-$C_3$-Alkylsulfinyl, $C_1$-$C_3$-Alkylsulfonyl, Dimethylaminosulfonyl, Diethylaminosulfonyl, N-Methoxy-N-methylaminosulfonyl, Phenyl, Phenoxy oder $C_1$-$C_3$-Alkoxycarbonyl steht und

$R^2$ für Wasserstoff, Fluor, Chlor, Brom, Methyl, Trifluormethyl oder Methoxy steht;

X für Wasserstoff, Methyl, Ethyl, Propyl, Methoxy, Ethoxy, Propoxy, Methylthio, Ethylthio, Propylthio oder Phenyl steht und

Y für Wasserstoff, Methyl, Ethyl, Propyl, Methoxy, Ethoxy, Propoxy, Methylthio, Ethylthio, Propylthio oder Phenyl steht.

**4.** Salze von sulfonylierten Triazincarbonsäureamiden der allgemeinen Formel (I) gemäß Ansprüchen 1, 2 und 3, gebildet

a) mit Protonensäuren, insbesondere mit Salzsäure, Schwefelsäure, Phosphorsäure, Methansulfonsäure, Benzol- oder p-Toluolsulfonsäure, oder Naphthalin-mono- oder di-sulfonsäuren, oder

b) mit Basen, insbesondere mit Natrium-, Kalium- oder Calcium-hydroxid, - hydrid, -amid oder -carbonat, Natrium- oder Kalium-$C_1$-$C_4$-alkanolaten, Ammoniak, $C_1$-$C_4$-Alkylaminen, Di-($C_1$-$C_4$-alkyl)-aminen oder Tri-($C_1$-$C_4$-alkyl)-aminen.

**5.** Verfahren zur Herstellung von sulfonylierten Triazincarbonsäureamiden der Formel (I) oder deren Salzen, dadurch gekennzeichnet, daß man Triazincarbonsäuren der Formel (II)

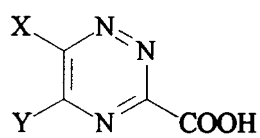

(II)

in welcher

X und Y die in Anspruch 1 angegebene Bedeutung haben,

oder reaktionsfähige Derivate von Verbindungen der Formel (II)

mit Sulfonsäureamiden der allgemeinen Formel (III)

$H_2N$-$SO_2$-$(A)_n$-R (III)

in welcher

n, A und R die in Anspruch 1 angegebene Bedeutung haben,

oder mit reaktionsfähigen Derivaten von Verbindungen der Formel (III)

gegebenenfalls in Gegenwart von Reaktionshilfsmitteln und gegebenenfalls in Gegenwart von Verdünnungsmitteln umsetzt und gegebenenfalls die so erhaltenen Produkte nach üblichen Methoden in Salze überführt.

**6.** Herbizide Mittel, gekennzeichnet durch einen Gehalt an mindestens einer Verbindung der Formel (I) gemäß Anspruch 1.

**7.** Verwendung von Verbindungen der allgemeinen Formel (I) gemäß Anspruch 1 zur Bekämpfung von unerwünschtem Pflanzenwachstum.

**8.** Verfahren zur Bekämpfung von Unkräutern, dadurch gekennzeichnet, daß man Verbindungen der allgemeinen Formel (I) gemäß Anspruch 1 auf die Unkräuter oder ihren Lebensraum einwirken läßt.

**9.** Verfahren zur Herstellung von herbiziden Mitteln, dadurch gekennzeichnet, daß man Verbindungen der allgemeinen Formel (I) gemäß Anspruch 1 mit Streckmitteln und/oder oberflächenaktiven Mitteln vermischt.

Europäisches Patentamt

# EUROPÄISCHER RECHERCHENBERICHT

Nummer der Anmeldung

EP 93 11 5264

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,Y | EP-A-0 353 640 (HOECHST AG)<br>7. Februar 1990<br>* Seite 11, Zeile 15 - Seite 11, Zeile 52; Ansprüche 1-7 * | 1-9 | C07D253/07<br>A01N43/707 |
| Y | EP-A-0 407 888 (BASF)<br>16. Januar 1991<br>* Seite 15, Zeile 56 - Seite 16, Zeile 29; Ansprüche 1-3 * | 1-9 | |
| A | WO-A-9 202 513 (FUJISAWA PHARMACEUTICAL CO., LTD.)<br>20. Februar 1992<br>* gesamtes Dokument * | 1-9 | |
| A | CHEMICAL ABSTRACTS, vol. 100, no. 25, 18. Juni 1984, Columbus, Ohio, US;<br>abstract no. 209754b,<br>G. SEITZ, S. DIETRICH 'Intramolecular (4+2)-cycloadditions of substituted triazines with inverse electron demand'<br>Seite 591 ;<br>* Zusammenfassung *<br>& Arch. Pharm. (Weinheim) 317 (4) 379-81 (1984) | 1-9 | |
| | | | RECHERCHIERTE SACHGEBIETE (Int. Cl.5) |
| | | | C07D<br>A01N |

Der vorliegende Recherchenbericht wurde für alle Patentansprüche erstellt

| Recherchenort | Abschlußdatum der Recherche | Prüfer |
|---|---|---|
| MUENCHEN | 19 JANUAR 1994 | HERZ C.P. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)